# EUROPÄISCHE PATENTSCHRIFT

(11) **EP 1 716 876 B1**
(45) Veröffentlichungstag und Bekanntmachung des Hinweises auf die Patenterteilung: **17.02.2010**
(21) Anmeldenummer: 05103460.1
(22) Anmeldetag: 27.04.2005
(51) Int. Cl.: A61M 3/02, F16K 24/04

(54) **Be- und entlüftetes Darmspülgerät**
Vented device for irrigation of the intestine
Dispositif ventilé pour irrigation d'intestin

(43) Veröffentlichungstag der Anmeldung: 02.11.2006
(73) Patentinhaber: Herrmann, Wolfgang, 63820 Eichelsbach (DE)
(72) Erfinder: Herrmann, Wolfgang, 63820 Eichelsbach (DE)
(74) Vertreter: Götz, Georg Alois

(56) Entgegenhaltungen:
- DE-A1- 4 232 777
- DE-A1- 4 429 068
- DE-U1- 29 606 767
- DE-U1-202004 015 358
- FR-A- 2 847 648

## Beschreibung

Die Erfindung betrifft ein Darmspülgerät mit einer Be- und Entlüftungsanordnung mit einer Darm-Zuflussleitung, welche mit einem menschlichen oder tierischen Darm zum Zuführen von Spülflüssigkeit verbindbar ist, und mit einer Darm-Abflussleitung, deren Eingang mit dem Ausgang des menschlichen oder tierischen Darms zum Abführen eines ausgespülten Darminhalts verbindbar ist.

Ent- oder belüftete Darmspülgeräte, insbesondere zur Anwendung im medizinisch-therapeutischen Bereich, sind seit langer Zeit bekannt (vgl. DE-U-296 10 226 oder DE-U-20 2004 015 358). Ein derartiges Gerät verfügt üblicherweise über eine Zuflussleitung, über die das Wasser unter geringem Druck mit Hilfe eines Katheters oder Spekulums in den Darm eingeleitet wird. Das Einspülen der Spülflüssigkeit wird normalerweise durch den Therapeuten mittels Massagen unterstützt. Des weiteren dienen die Massagen dazu, Verkrustungen und Ablagerungen zu lösen, damit diese mit dem Abfluß der Spülflüssigkeit aus dem Darm entfernt werden können. Der Katheter oder das Spekulum verfügt über einen Anschluß für eine Abflußleitung, die zurück zum Gerätegehäuse geführt wird und dort in eine Sichtröhre zur Kontrolle der abfließenden Spülflüssigkeit geleitet wird. Ein Absperrorgan zur Stauung des Wassers ist üblicherweise im Verlauf der Abflußleitung vor oder nach der Sichtröhre angeordnet. Nach der Sichtröhre wird die Abflußleitung fortgesetzt und an einen bauseits gestellten Abfluß angeschlossen. Über diesen Abfluß wird die abgeführte Spülflüssigkeit in die Kanalisation abgeleitet.

Weiterhin verfügt ein derartiges Gerät üblicherweise über einen Kalt- und einen Warmwasserzulauf, aus denen mit Hilfe eines Temperaturreglers ein für den Patienten angenehm temperiertes Wassergemisch eingestellt werden kann. Daneben ergibt es einen Druckregler, mit dem auch der Druck auf die Bedürfnisse des Patienten angepaßt werden kann. Zur Überprüfung von Temperatur und Druck des Wassers verfügen gattungsgemäße Geräte weiterhin über eine Temperatur- und eine Druckanzeige.

Bei der Behandlung mit einem derartigen Darmspülgerät kann es während der Behandlung beim Patienten durch Luft im Darm, insbesondere bei Patienten, die wegen Blähungen in Behandlung sind, zu einem unangenehmen Druckgefühl kommen. Zusätzlich behindert diese Luft das Einspülen der Spülflüssigkeit und verlängert so den Behandlungsvorgang. Verstärkt wird diese Problematik noch durch mit der Spülflüssigkeit in den Darm des Patienten eingeführte Luft. Die Therapeuten behelfen sich während der Behandlung oft durch kurzes, zeitweises Abziehen des Abflußschlauches, um derart einen Druckausgleich und somit eine Entlüftung durchzuführen. Nachteilig ist hierbei aber, dass sich der Patient zum Zwecke der Entlüftung in eine Seitenlage drehen muss, was angesichts des in den After eingeführten Spekulums insbesondere für alte und/oder kranke Menschen beschwerlich und auch peinlich ist. Auch lassen sich bei einem derartigen, manuellem Druckausgleich der Austritt von übel riechenden Gasen und kleinen Mengen Spülflüssigkeit nicht vermeiden. Dies führt zum einen zu einer Geruchsbelästigung während der Behandlung, zum anderen ist ein derartiger Austritt von verunreinigtem Spülwasser auch hygienisch bedenklich. Durch die nicht vermeidbare Verschmutzung der Liege erhöht sich daneben auch noch der Reinigungsaufwand was die Personalkosten für die Behandlung in die Höhe treibt. Schließlich verlängert sich die Behandlungsdauer durch die Verzögerungen beim manuellen Entlüften erheblich, so dass die Effizienz der Behandlungsmethode sinkt und die Kosten somit steigen. Wenn nun ausreichend Flüssigkeit in den Darm eingeleitet wurde, wird bei der Behandlung das Absperrorgan der Abflußleitung geöffnet, und durch die Sogwirkung des tiefer liegenden Abflusses entleert sich der Darm, wobei die Ablagerungen und Verkrustungen in der Spülflüssigkeit mit geführt werden. Hierbei kann aber das Problem auftreten, dass durch feste Bestandteile in der Spülflüssigkeit die Abflußleitung zumindest temporär verstopft wird, so dass das mit Kotanteilen angereicherte Wasser nur schlecht oder gar nicht abfließen kann. Eine Verbesserung der Abflußleitung einer Darmspülvorrichtung ist also wünschenswert.

Das Problem, dass es während einer Darmspülung sehr häufig vorkommt, dass die mit den Darmbestandteilen belastete, vom Darmspülgerät abgesaugte Flüssigkeit beispielsweise in einem Darminhalt-Sichtrohr stehen bleibt und so der Absaugvorgang unterbrochen wird, wird in der Gebrauchsmusterveröffentlichung DE-U-20 2004 016 919 angegangen. Dazu wird eine Vorrichtung zur Belüftung des Darmspülgeräts mittels eines Belüftungsventils vorgeschlagen, welches mit der zum Sichtrohr führenden Abflussleitung gekoppelt ist. Wenn nicht belüftet werden muss, befindet sich das Ventil in der Ausgangsstellung, wobei der Ventilkörper mittels Federdruck dichtend gegen einen Ventilsitz gedrückt wird. Kommt es zu einem Stillstand des Spülvorganges, der durch die Bedienperson von außen im Sichtrohr erkennbar ist, so braucht sie nur kurz den Bedienknopf des Belüftungsventils zu drücken, wodurch letzteres gegen Federdruck geöffnet wird. Aufgrund einer Sogwirkung wird dem Stau in der Abflussleitung Luft zugeführt, ohne dass übel riechende Gase austreten. In der offenen Stellung des Ventils, bei der dessen Bedienknopf gedrückt ist, lässt sich allerdings die Gefahr nicht ausschließen, dass über den Ventildurchgang verschmutztes Wasser bzw. Darminhalte enthaltende Flüssigkeit austreten könnten. Diese Gefahr ist z. B. dann gegeben, wenn der Bedienknopf fehlerhaft bzw. versehentlich gedrückt wird.

Der Erfindung liegt die Aufgabe zugrunde, bei einem Darmspülgerät die Funktions- und Bediensicherheit zu erhöhen. Zur Lösung wird das im Anspruch 1 angegebene Darmspülgerät mit einer entsprechenden Be- und Entlüftungsanordnung vorgeschlagen. Vorteilhafte, optionale Ausgestaltungen ergeben sich aus den abhängigen Ansprüchen.

Mit dem erfindungsgemäß ausgangsseitig in der Darm-Abflussleitung angeordneten Absperrorgan lässt sich ein Stauen der Darmspülflüssigkeit bis in den Dickdarm hinein herbeiführen. Bei dieser Stauung wird vielfach unter anderem auch die Luft des Patienten oder auch die Luft aus dem Schlauchsystem des Darmspülgeräts mitgestaut. Dies kann zu Überblähungen des Patienten führen. Der Abhilfe dient die Entlüftungskomponente der Be- und Entlüftungseinrichtung. Kommt es beim Stau- und Entlüftungsvorgang zu einem Hochschwappen von Flüssigkeit mit ausgespültem Darminhalt in die Leitung zum Entlüftungsventil, wird die Gefahr einer Umgebungsverschmutzung durch den automatischen Schließmechanismus beseitigt, der in der Be- und Entlüftungseinrichtung integriert ist und ab einer bestimmten Druckschwelle entsprechend dem unzulässigen Ansteigen des Flüssigkeitspegels anspricht, nachdem vorher die Luft des Spülsystems ausgeströmt ist. Ansonsten ist die Be- und Entlüftungseinrichtung in einer stets bidirektional geöffneten Dauerstellung, welche ein automatisches Entlüften, also ohne manuellen Eingriff des Therapeuten, ermöglicht.

Zur zuverlässigen Abfuhr von Luft oder Gasen aus dem Abflusssystem des Darmspülgeräts besteht eine vorteilhafte Ausbildung der Erfindung darin, den Verbindungskanal zur Be- und Entlüftungseinrichtung an der Stelle der Abflussleitung anzuschließen, welche am höchsten liegt. Dabei wird von dem an sich bekannten Effekt Gebrauch gemacht, dass Luft- oder Gasblasen in Flüssigkeiten das Bestreben haben, nach oben zu steigen.

Die Betriebszuverlässigkeit lässt sich erhöhen, und die Gefahr von Geruchsbelästigungen vermindern, dadurch dass erfindungsgemäß die Be- und Entlüftungseinrichtung innerhalb eines Gerätegehäuses angeordnet ist und insbesondere darin vollständig abgeschlossen umgeben ist.

Um das Austreten von Spülflüssigkeit zuverlässig zu verhindern, ist es wichtig, dass die Vorrichtung zur Entlüftung der Abflußleitung automatisch arbeitet, dass heißt, sie muss eine Verbindung zur Raumluft für die Herstellung des Druckausgleichs möglichst permanent zur Verfügung stellen, gleichzeitig aber bei der Gefahr von austretendem Wasser umgehend die Öffnung verschließen. Dies wird erfindungsgemäß dadurch erreicht, dass ein Ventil zum Einsatz kommt, das für Luft offen ist, aber aufsteigendes Wasser sofort stoppt.

Für die Verbesserung der reduzierten Abflußleistung bei Kotanteilen in der abfließenden Spülflüssigkeit kann eine Erhöhung der Abflußmenge beziehungsweise eine Verbesserung des Abflußverhaltens erfindungsgemäß dadurch erreicht werden, dass der Abflußleitung beim Ablassen der Spülflüssigkeit Luft zugeführt wird. Wie an sich bekannt ist, erhöht das Einbringen von Luft beziehungsweise das Belüften von Rohrleitungen durch Verminderung der Wirbelbildung zu einer erhöhten Abflußkapazität. Gemäß einer Erfindungsalternative wird daher eine Vorrichtung zur Belüftung der Abflußleitung vor dem Absperr- und Regelorgan an der Abflußleitung angeordnet. Bei Öffnung des Absperr- beziehungsweise Regelorgans entsteht an der Vorrichtung zur Belüftung der Abflußleitung ein Sog, der zum Mitreißen von kleinen Luftbläschen führt und somit zur Verbesserung der Abflußleistung beiträgt. Zur Vermeidung austretender Spülflüssigkeit ist auch hierbei die Belüftungsvorrichtung derart ausgelegt, dass bei in die Belüftungsvorrichtung aufsteigende Wasser diese sperrt, während sie ansonsten für die Zuführung von Luft geöffnet ist. Diese Sperrung erfolgt auch wieder automatisch und erfordert keine Interaktion durch den Therapeuten.

In der Erfindung wird ein Darmspülgerät vorgeschlagen, bei dem Vorrichtungen zur Be- und Entlüftung kombiniert integriert sind. Auf diese Art und Weise können die Vorteile von Be- und Entlüftung der Abflußeinrichtung unter Erzielung von Synergieeffekten kombiniert werden. Zur Realisierung dieser kombinierten Erfindungsgedanken besteht eine vorteilhafte Ausbildung darin, ein Be- und Entlüftungsventil zu verwenden, das mit einem gegen einen Ventilsitz eines durchströmbaren Ventilgehäuses per Fluiddruck verstellbaren Schwimmkörper realisiert ist. In weiterer Ausbildung dieser Variante wird das Be- und Entlüftungsventil im oder am Spülgerät so angeordnet, dass der Schwimmkörper aufgrund seines Eigengewichts vom Ventilsitz selbsttätig wegverstellbar ist. Alternativ ist auch der Einsatz von Federelementen zur Beibehaltung der offenen Ventilstellung denkbar. Ein derartiges automatisches Be- und Entlüftungsventil ist aus der Gebrauchsmusterveröffentlichung DE-U-296 06 767 bekannt. Als Be- und Entlüftungsventile ebenso geeignet sind an sich bekannte Rohrbelüfter, beispielsweise von der Firma Seppelfricke Armaturen GmbH & Co., D-45881 Gelsenkirchen unter der Modell-Nr. 8171 und 8172 - Bauform D.

In einer weiteren vorteilhaften Weiterbildung der Erfindung wird zur Erhöhung der Sicherheit vor austretender Spülflüssigkeit um die Vorrichtung zum Be- und/oder Entlüften der Abflußleitung zusätzlich ein Auffang- oder Überlaufbehälter angebracht. So kann im Falle von geringen austretenden Mengen der Spülflüssigkeit zuverlässig verhindert werden, dass diese unkontrolliert ins Gehäuse der Darmspülvorrichtung gelangen. Hierdurch wird die Betriebssicherheit des Gerätes noch einmal beträchtlich erhöht und die Anwendung für den Therapeuten vereinfacht. Von diesem Überlaufbehälter kann in vorteilhafter Weise eine Leitung zur Abflußleitung geführt werden, so dass die eventuell ausgetretenen, geringen Spülflüssigkeitsmengen zuverlässig in Richtung Kanalisation abgeführt werden. Diese Leitung zur Abflußleitung kann vorteilhaft hinter dem Absperr- und Regelorgan angeordnet werden, so dass auch bei geschlossenem Absperr- beziehungsweise Regelorgan eine zuverlässige Abführung der eventuell ausgetretenen Spülflüssigkeit gewährleistet werden kann.

Die Vorrichtungen zur Be- und Entlüftung sind im erfindungsgemäßen Darmspülgerät integriert ausgeführt. Bei einer derartigen Ausführung ergänzen sich in überraschender Weise die Vorteile und Eigenschaften der automatischen selbsttätigen Be- und Entlüftung. So ist es bei der kombinierten Be- und Entlüftung zwar möglich, dass im Rahmen der Entlüftung des Darmes beziehungsweise der Abflußleitung unangenehme Gerüche austreten und ins Innere des Darmspülgeräts entweichen, da aber die Belüftungsfunktion durch die mitgerissene und somit entfernte Luft eine gewisse Saugwirkung entwickelt, werden unangenehme Gerüche noch vor dem Austreten aus dem Gehäuse des Darmspülungsgeräts wieder eingesaugt und derart entfernt. Auf diese Weise können die entstehenden unangenehmen Gerüche automatisch entfernt werden, noch bevor sie zu einer Belästigung von Patient und Therapeut führen.

Die folgenden Zeichnungen zeigen eine bevorzugte, beispielhafte Ausführungsform der Erfindung, wobei die Erfindung durch die Zeichnungen in keiner Weise eingeschränkt werden soll und viele alternative Ausführungsformen und (Unter-) Kombinationen, die gleichwohl unter denselben Erfindungsgedanken fallen, denkbar sind. Hierbei zeigen:
- Figur 1: schematisch ein erfindungsgemäßes Spülgerät mit auf Patientenliege liegendem Patient;
- Figur 2: eine schematische Funktionsdarstellung des Inneren des Darmspülgeräts nach Figur 1;
- Figur 3: teilweise axial geschnitten ein Be- und Entlüftungsventil, das zum Einsatz in einem erfindungsgemäßen Darmspülgerät geeignet ist.

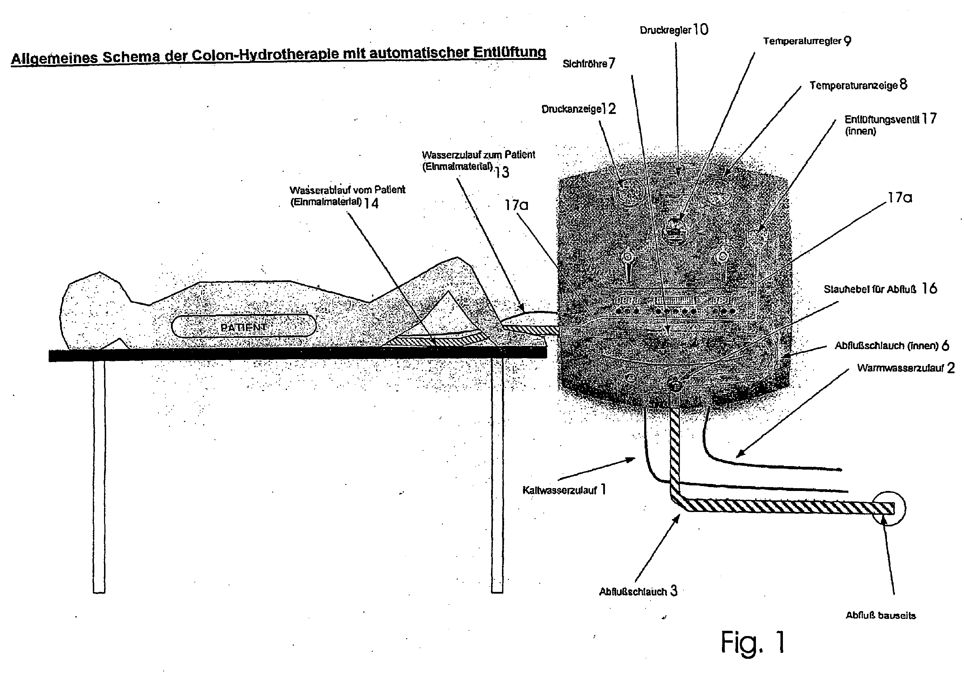

Figur 1 zeigt schematisch den Aufbau eines Behandlungsraums mit einem erfindungsgemäßen Darmspülgerät. Das Darmspülgerät wird ungefähr auf gleicher Höhe mit dem Patient auf der Behandlungsliege montiert und über die Zuläufe 1 und 2 an Kalt- beziehungsweise Warmwasser angeschlossen. Ein Abflußschlauch 3 wird vom Abwasserausgang des Darmspülgeräts zu einem bauseits gestellten Abfluß geführt. Das über die Zuläufe 1 und 2 zugeführte Wasser wird mit dem Temperaturregler 9 auf eine für den Patienten angenehme Wassertemperatur eingestellt, die mittels der Temperaturanzeige 8 kontrolliert werden kann. Über den Druckregler 10 kann der Einspüldruck für den Patienten komfortabel gestaltet werden und über die Druckanzeige 12 ebenfalls kontrolliert werden. Durch den Wasserzulauf 13, der aus Einmalmaterial besteht, wird das Wasser zum Patienten zugeführt. Wenn der Darm des Patienten mittels unterstützender Massage ausreichend gefüllt und gesäubert ist, wird der Zufluß geschlossen und der Abfluß mittels dem Stauhebel 16 geöffnet. Das im Darm befindliche Wasser fließt über den Wasserablauf 14, der ebenfalls aus Einmalmaterial besteht, zum inneren Abflußschlauch 6 und von dort über die Sichtröhre 7 zum Abflußschlauch 3 und über den bauseits gestellten Abfluß 18a in die Kanalisation. Zwischen dem Anschluß des Wasserablaufs 14 am Gerät und der Sichtröhre 7 befindet sich im Abflußschlauch 6 (im Bild gestrichelt dargestellt) die Leitung 17a zur Entlüftungsvorrichtung 17. Eventuell auftretende Luft im Darm und/oder Abflußschlauch kann über dieses Entlüftungsventil 17 entweichen.

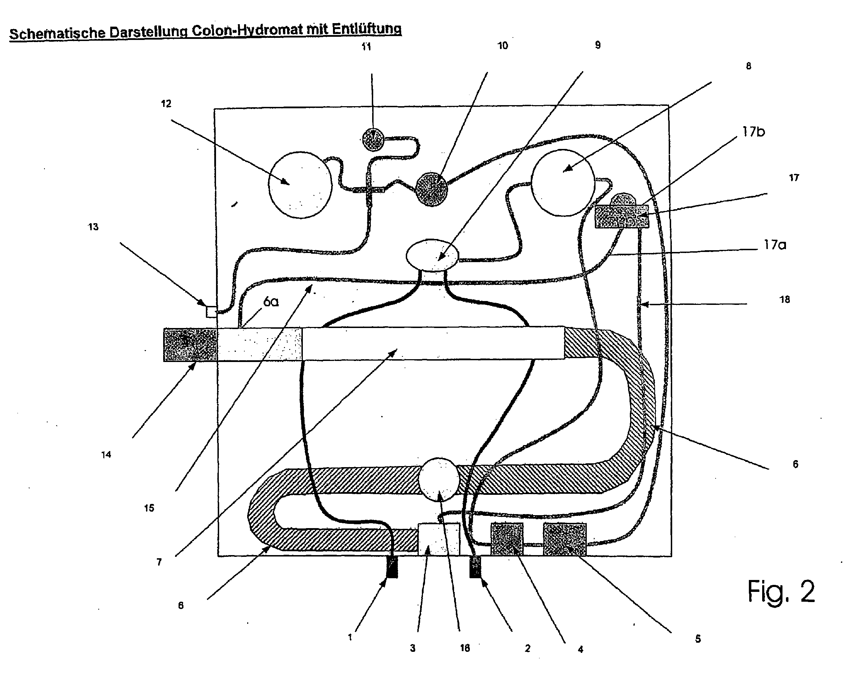

Figur 2 zeigt schematisch den inneren Aufbau eines erfindungsgemäßen Darmspülgeräts. Nachdem das Wasser über die Zuläufe 1 und 2 in das Gerät gelangt ist, wird es von dort mittels Leitungen zum Temperaturregler 9 geführt. Vom Temperaturregler 9 wird das Wasser ebenfalls über eine Leitung zur Temperaturanzeige 8 geführt, mittels derer der Therapeut die Temperatur für den Patienten angenehm einstellen kann. Von der Temperaturanzeige 8 wird das Wasser über eine weitere Leitung zu einem Druckminderer 4 geleitet, der den im Hausnetz vorhandenen Wasserdruck auf den maximalen Behandlungsdruck abregelt. Direkt hinter dem Druckminderer ist ein Überdruck-Absperrventil 5 in der Leitung angeordnet, dass als zusätzliche Sicherheitsmaßnahme bei Überdruck die Leitung zum Patienten unterbricht. Von diesem Überdruckventil 5 führt eine weitere Leitung zum Druckregler 10, mit dem auch der Einspüldruck für den Patienten angenehm gestaltet werden kann. Nach dem Druckregler 10 sind mittels Abzweigungen eine Druckanzeige 12 sowie ein Druckwächter 11 als weitere Sicherheitsmaßnahme gegen Überdruck angeordnet. Von der Druckregelungsgruppe wird das Wasser über eine weitere Leitung zum Anschluß 13 geführt, an dem die Zuflussleitung zum Patienten angeschlossen werden kann. Am Anschluß 14 wird die vom Patienten kommende Abflußleitung angeschlossen. Im darauffolgenden Abschnitt des inneren Abflußschlauchs 6 ist die Zuleitung zum Be- beziehungsweise Entlüftungsventil 17 in Strömungsrichtung noch vor der Sichtröhre 7 angeschlossen. Das Be- beziehungsweise Entlüftungsventil 17 ist mit einem Überlauf beziehungsweise Auffangbehälter 17b versehen, der eventuell austretende Spülflüssigkeit, die mit Kotanteilen verunreinigt ist, aufnehmen kann. Von diesem Auffangbehälter führt eine weitere Leitung 18 zum Abflußanschluß 3, der über den Abflußschlauch mit dem bauseits gestellten Abfluß 18a verbunden ist. An dem Bereich, in dem das Ventil 17 mit dem inneren Abflußschlauch 6 angeschlossen ist, befindet sich die Sichtröhre 7, mittels derer der Therapeut die ausgespülten Bestandteile des Spülwassers überprüfen und kontrollieren kann. Nach der Sichtröhre 7 führt der innere Abflußschlauch 6 über das Absperr- und Regelorgan 16 zum Abflußanschluß 3 oder zum Abfluss bauseits 18a.

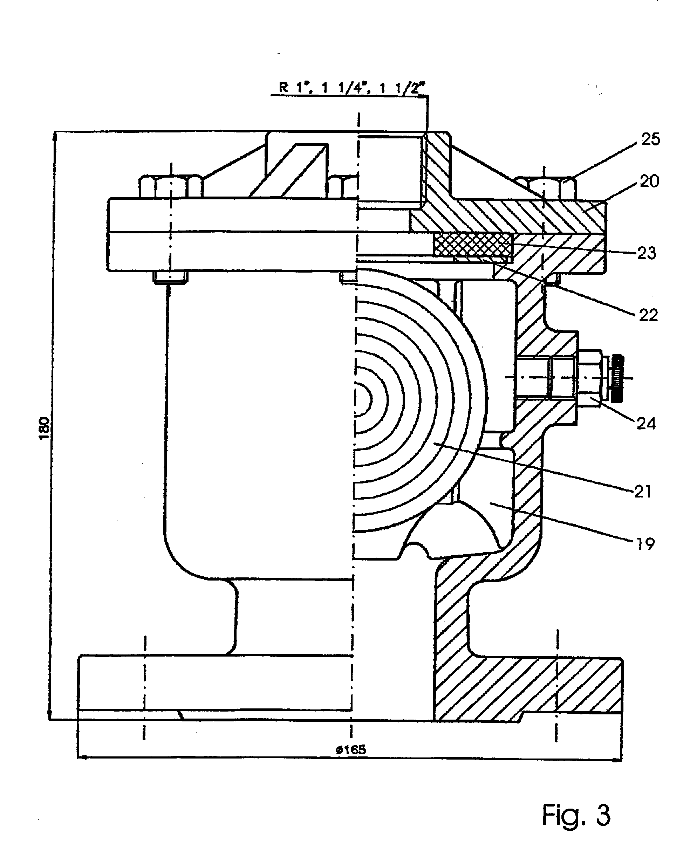

Figur 3 zeigt ein Be- und Entlüftungsventil, das in einer erfindungsgemäßen Darmspülmaschine eingesetzt werden kann. Dieses Ventil verfügt über das Ventilgehäuse 19, das zum Anschluß an eine Rohrleitung ausgebildet ist. Anschließend an den in der Zeichnung unten befindlichen Anschlußbereich befindet sich ein verbreiteter Umströmungsbereich, in dem sich ein Kugelschwimmer 21 befindet. Das Gehäuse 19 des Be- und Entlüftungsventils ist auf der oberen Seite offen, damit der Kugelschwimmer 21 problemlos in den Umströmungsbereich eingebracht werden kann. Nach Einbringung des Kugelschwimmers wird auf die obere Öffnung des Ventilgehäuses 19 ein Deckel 20 geschraubt. Die Verschraubung von Deckel 20 wird mit Hilfe der Schrauben 25 durchgeführt. Deckelteil 20 besitzt in der Mitte eine Öffnung, durch die Luft aus dem Inneren des Ventilgehäuses 19 entweichen kann. Um die Öffnung im Ventildeckel 20 sind auf Seite des Umströmungsbereichs im Ventilkörper 19 eine Unterlegscheibe 22 sowie eine Gummidichtscheibe 23 eingeschraubt. Dieses Be- und Entlüftungsventil wird im Gehäuse des Spülgeräts zweckmäßig derart montiert, dass sich der Deckelteil 20 oben befindet. Der Kugelschwimmer 21 befindet sich deshalb durch die Schwerkraft am unteren Ende des Umströmungsbereichs des Ventilgehäuses 19. Das Gewicht des Kugelschwimmers 21 wird dabei so gewählt, dass die ausströmende Luft in der Regel nicht in der Lage ist, ihn gegen die Schwerkraft zur Gummidichtscheibe 23 zu pressen. Wenn nun im unterhalb des Be- und Entlüftungsventils liegenden Rohr Wasser aufsteigt, so schwimmt der Kugelschwimmer 21 auf und preßt sich gegen die Gummidichtscheibe 23. Hierdurch wird das Be- und Entlüftungsventil für das Wasser undurchlässig und sperrt gegen das austretende Wasser. Jedenfalls wenn der Kugelschwimmer 21 nicht gegen die Gummidichtstelle 23 gedrückt ist, kann das Ventil von Luft bidirektional, d. h. sowohl zum Be- als auch zum Entlüften durchströmt werden.

Selbstverständlich können auch anders gebaute Be- und Entlüftungsventile in einem erfindungsgemäßen Darmspülgerät zum Einsatz kommen. Zweckmäßig ist dabei immer eine automatische Betätigung wenigstens bei aufsteigendem Wasser.

### Bezugszeichenliste

- 1: Kaltwasserzulauf
- 2: Warmwasserzulauf
- 3: Abflussanschluss
- 4: Druckminderer für max. Behandlungsdruck
- 5: Absperrventil bei Überdruck
- 6: Abflussschläuche (innen)
- 6a: Kopplungsstelle
- 7: Sichtröhre
- 8: Temperaturanzeige
- 9: Temperaturregler
- 10: Druckregler für Behandlungswasser-Zulauf
- 11: Druckwächter für Überdruck (250 mbar)
- 12: Druckanzeige für Behandlungsdruck
- 13: Anschluss für Behandlungswasser (Richtung Patient)
- 14: Anschluss für Abwasserschlauch (vom Patient kommend)
- 15: Schlauch von Sichtröhre zum Entlüftungsventil
- 16: Absperrhebel, um Patientenwasser zu stauen
- 17a: Leitung zum Entlüftungsventil
- 17: Entlüftungsventil mit Auffangbehälter
- 17b: Auffangbehälter
- 18: Schlauch vom Auffangbehälter zum Abflussanschluss
- 18a: Abfluss bauseits
- 19: Ventilgehäuse
- 20: Deckel
- 21: Kugelschwimmer
- 22: Unterlegscheibe
- 23: Gummidichtscheibe

## Patentansprüche

1. Darmspülgerät mit einer Be- und Entlüftungsanordnung, mit einer Darm-Zuflussleitung (13), welche mit einem menschlichen oder tierischen Darm zum Zuführen von Spülflüssigkeit verbindbar ist, und mit einer Darm-Abflussleitung (6), deren Eingang mit dem Ausgang des menschlichen oder tierischen Darms zum Abführen eines ausgespülten Darminhalts verbindbar ist, **dadurch gekennzeichnet, dass** die Darm-Abflussleitung (6), die als innere Darm-Abflussleitung (6) im Inneren eines Gerätegehäuses verläuft, mit einem ausgangsseitig zum Stauen von Flüssigkeit im Darm angeordneten Absperrorgan (16) verschließbar und in ihrem Abschnitt zwischen Eingang und Ausgang mit wenigstens einer Be- und Entlüftungseinrichtung (17, 17a) gekoppelt ist, die innerhalb des Gerätegehäuses angeordnet und zum selbsttätigen Schließen ab einer bestimmten Druckschwelle in der Darm-Abflussleitung (6) ausgebildet ist.

2. Darmspülgerät nach Anspruch 1, wobei die Darm-Abflussleitung (6) gegenüber einer Horizontalen abweichend verläuft, **dadurch gekennzeichnet, dass** sich die Kopplungsstelle (6a) der Darm-Abflussleitung (6) für die Be- und Entlüftungseinrichtung (17, 17a) im gegenüber der Horizontalen am höchsten gelegenen Bereich der Darm-Abflussleitung (6) befindet.

3. Darmspülgerät nach einem der vorangehenden Ansprüche, **dadurch gekennzeichnet, dass** die Be- und Entlüftungseinrichtung (17, 17a) als zum Be- und Entlüften bidirektional offenes Ventil (19 - 25) ausgebildet ist, das einerseits mit der Umgebungsluft in Verbindung steht und andererseits mit dem Druck in der Darm-Abflussleitung (6) zum automatischen Schließen ab einer bestimmten Druckschwelle gekoppelt ist.

4. Darmspülgerät nach Anspruch 3, **dadurch gekennzeichnet, dass** das Ventil (19 - 25) mit einem gegen einen Ventilsitz (20, 22, 23) eines durchströmbaren Ventilgehäuses (19) per Fluiddruck verstellbaren Schwimmkörper (21) realisiert ist, der mittels Federdruck und/oder seines Eigengewichts vom Ventilsitz (20, 22, 23) weg verstellbar ist.

5. Darmspülgerät nach einem der vorangehenden Ansprüche, **dadurch gekennzeichnet, dass** die Be- und Entlüftungseinrichtung (17, 17a) mit einem Rohrbelüfter realisiert ist.

6. Darmspülgerät nach einem der vorangehenden Ansprüche, **dadurch gekennzeichnet, dass** die Be- und Entlüftungseinrichtung (17, 17a) mit einem Auffangbehälter (17b) für austretende Flüssigkeit versehen ist.

7. Darmspülgerät nach Anspruch 6, **dadurch gekennzeichnet, dass** der Auffangbehälter (17b) mit einer Abführleitung (18) versehen ist, die ganz oder teilweise vertikal zur Darm-Abflussleitung (6) verläuft und dort einmündet.

8. Darmspülgerät nach Anspruch 7, **dadurch gekennzeichnet, dass** die Abführleitung (18) in Strömungsrichtung nach dem Absperrorgan (16) in den Ausgangsbereich der Darm-Abflussleitung (6) einmündet.

## Claims

1. An intestinal irrigation device with an aeration and ventilation system, having an intestinal inflow line (13) which can be connected to a human or animal intestine for delivering irrigating fluid, and having an intestinal outflow line (6) the inlet of which can be connected to the outlet of the human or animal intestine in order to discharge an irrigated intestinal content, **characterised in that** the intestinal outflow line (6), which extends as an inner intestinal outflow line (6) within a device housing, is coupled closeably with a blocking component (16) disposed on the outlet side in order to retain fluid within the intestine and in its section between the inlet and the outlet to at least one aeration and ventilation device (17, 17a) which is disposed within the device housing and is designed to provide automatic closure from a specific pressure threshold onwards in the intestinal outflow line (6).

2. The intestinal irrigation device according to Claim 1, the intestinal outflow line (6) extending divergently in relation to a horizontal, **characterised in that** the coupling point (6a) of the intestinal outflow line (6) for the aeration and ventilation device (17, 17a) is located at the highest region of the intestinal outflow line (6) in relation to the horizontal.

3. The intestinal irrigation device according to any of the preceding claims, **characterised in that** the aeration and ventilation device (17, 17a) is in the form of a bi-directionally open valve (19 - 25) for aerating and venting, which on the one hand is connected to the ambient air, and on the other hand is coupled to the pressure in the intestinal outflow line (6) in order to automatically close from a specific pressure threshold onwards.

4. The intestinal irrigation device according to Claim 3, **characterised in that** the valve (19 - 25) is produced with a floating body (21) that can be adjusted towards a valve seat (20, 22, 23) of a valve housing (19) through which fluid can flow depending on the fluid pressure, and which can be adjusted away from the valve seat (20, 22, 23) by means of spring pressure and/or its own weight.

5. The intestinal irrigation device according to any of the preceding claims, **characterised in that** the aeration and ventilation device (17, 17a) is produced with a tube aerator.

6. The intestinal irrigation device according to any of the preceding claims, **characterised in that** the aeration and ventilation device (17, 17a) is provided with a collection container (17b) for escaping fluid.

7. The intestinal irrigation device according to Claim 6, **characterised in that** the collection container (17b) is provided with a discharge line (18) which extends totally or partially vertically to the intestinal outflow line (6) and discharges inwardly here.

8. The intestinal irrigation device according to Claim 7, **characterised in that** the discharge line (18) discharges in the direction of flow after the blocking device (16) into the outlet region of the intestinal outflow line (6).

## Revendications

1. Appareil de lavage d'intestin avec un aménagement d'acheminement et d'évacuation d'air, comprenant un tuyau (13) d'acheminement à l'intestin, qui peut être relié à un intestin humain ou animal pour acheminer un liquide de lavage, et un tuyau (6) d'évacuation de l'intestin, dont l'entrée peut être reliée à la sortie de l'intestin humain ou animal pour évacuer un contenu d'intestin expulsé, **caractérisé en ce que** le tuyau d'acheminement à l'intestin (6) qui passe, en tant que tuyau interne (6) d'acheminement à l'intestin, à l'intérieur d'un boîtier d'appareil, peut être fermé avec un organe d'arrêt (16) aménagé, côté sortie, pour retenir le liquide dans l'intestin et est raccordé, dans sa section entre l'entrée et la sortie, à au moins un dispositif d'acheminement et d'évacuation d'air (17, 17a), qui est aménagé à l'intérieur du boîtier d'appareil et est conformé pour une fermeture automatique à partir d'un certain seuil de pression dans le tuyau (6) d'évacuation de l'intestin.

2. Appareil de lavage d'intestin selon la revendication 1, dans lequel le tuyau (6) d'évacuation de l'intestin s'étend en s'écartant de l'horizontale, **caractérisé en ce que** le point de raccordement (6a) du tuyau (6) d'évacuation de l'intestin destiné au dispositif d'acheminement et d'évacuation d'air (17, 17a) se trouve dans la zone la plus élevée, par rapport à l'horizontale, du tuyau (6) d'évacuation de l'intestin.

3. Appareil de lavage d'intestin selon l'une quelconque des revendications précédentes, **caractérisé en ce que** le dispositif d'acheminement et d'évacuation d'air (17, 17a) se présente sous la forme d'une soupape (19 à 25) à ouverture bidirectionnelle pour l'acheminement et l'évacuation d'air, laquelle soupape est, d'une part, en communication avec l'air ambiant et est, d'autre part, raccordée à la pression dans le tuyau (6) d'évacuation de l'intestin pour une fermeture automatique à partir d'un certain seuil de pression.

4. Appareil de lavage d'intestin selon la revendication 3, **caractérisé en ce que** la soupape (19 à 25) est réalisée avec un corps flottant (21) réglable par pression de fluide contre un siège de soupape (20, 22, 23) d'un boîtier de soupape (19) pouvant être traversé et qui est réglable par éloignement du siège de soupape (20, 22, 23) à l'aide d'une pression de ressort et/ou sous l'influence de son propre poids.

5. Appareil de lavage d'intestin selon l'une quelconque des revendications précédentes, **caractérisé en ce que** le dispositif d'acheminement et d'évacuation d'air (17, 17a) est réalisé avec un aérateur tubulaire.

6. Appareil de lavage d'intestin selon l'une quelconque des revendications précédentes, **caractérisé en ce que** le dispositif d'acheminement et d'évacuation d'air (17, 17a) est équipé d'un récipient collecteur (17b) pour le liquide sortant.

7. Appareil de lavage d'intestin selon la revendication 6, **caractérisé en ce que** le récipient collecteur (17b) est pourvu d'un tuyau d'évacuation (18), qui s'étend, en totalité ou en partie, verticalement au tuyau (6) d'évacuation de l'intestin et y débouche.

8. Appareil de lavage d'intestin selon la revendication 7, **caractérisé en ce que** le tuyau d'évacuation (18) débouche dans le sens d'écoulement après l'organe d'arrêt (16) dans la zone de sortie du tuyau (6) d'évacuation de l'intestin.
